# FASCICULE DE BREVET EUROPEEN

(11) **EP 3 696 203 B1**
(45) Date de publication et mention de la délivrance du brevet: **23.08.2023**
(21) Numéro de dépôt: 20157465.4
(22) Date de dépôt: 14.02.2020
(51) Int. Cl.: C08F 292/00, B22F 1/102, B22F 1/16, H01G 4/30, H01G 4/10, H01G 4/20

(54) **PROCEDE DE FABRICATION DE NANOPARTICULES HYBRIDES METAL/POLYMERE A FAIBLE DISTRIBUTION DE TAILLES PAR POLYMERISATION EN MINI-EMULSION**
HERSTELLUNGSVERFAHREN VON HYBRIDEN METALL-/POLYMER-NANOPARTIKELN MIT GERINGER GRÖSSENVERTEILUNG DURCH POLYMERISATION IN MINI-EMULSIONEN
METHOD FOR MANUFACTURING HYBRID METAL/POLYMER NANOPARTICLES WITH LOW DISTRIBUTION OF SIZES BY POLYMERISATION IN MINI-EMULSION

(30) Priorité: 18.02.2019 FR 1901599
(43) Date de publication de la demande: 19.08.2020
(73) Titulaire: Commissariat à l'énergie atomique et aux énergies alternatives, 75015 Paris (FR)
(72) Inventeur: VIALA, Bernard, 38054 GRENOBLE CEDEX 09 (FR)
(74) Mandataire: Brevalex

(56) Documents cités:
- TAKACS H ET AL: "Non-conductive ferromagnetic carbon-coated (Co, Ni) metal/polystyrene nanocomposites films", JOURNAL OF APPLIED PHYSICS, AMERICAN INSTITUTE OF PHYSICS, US, vol. 119, no. 9, 7 mars 2016 (2016-03-07), XP012205780, ISSN: 0021-8979, DOI: 10.1063/1.4942862 [extrait le 1901-01-01]
- H?l?ne Takacs ET AL: "Non-conductive ferromagnets based oncore double-shell nanoparticles forradio-electric applications Background", , 2016, XP055345832, DOI: 10.1186/s40064-016-2099-3 Extrait de l'Internet: URL:http://download.springer.com/static/pd f/395/art%3A10.1186%2Fs40064-016-2099-3.pd f?originUrl=http://springerplus.springerop en.com/article/10.1186/s40064-016-2099-3&t oken2=exp=1487088018~acl=/static/pdf/395/a rt%253A10.1186%252Fs40064-016-2099-3.pdf*~ hmac=fb8b50763a25052fb4b67c6f88f72edec9941 44cea7e94c [extrait le 2017-02-14]

## Description

### DOMAINE TECHNIQUE

La présente invention se rapporte au domaine des matériaux nanocomposites métal-polymère de dimensions déterminées, et notamment à la fabrication de nanoparticules à structure coeur/coquille.

Plus spécifiquement, elle se rapporte à un procédé de fabrication de nanoparticules à faible distribution de taille comprenant un coeur métallique recouvert par une couche de matériau polymère.

La présente invention trouve des applications dans le domaine de l'électromagnétisme, de l'optique à la conversion d'énergie, ou encore de la biologie.

### ÉTAT DE LA TECHNIQUE ANTÉRIEURE

Les nanoparticules hybrides métal-polymère sont utilisées dans de nombreux domaines : le biomédical, les revêtements (anti-microbien et anti-fongique par exemple), la peinture, l'optique, l'électronique, le magnétisme et les systèmes catalytiques.

Les nanoparticules hybrides métal-polymère sont des nanoparticules comprenant un coeur métallique enrobé d'un polymère.

Classiquement, pour fabriquer de tels matériaux, des nanoparticules métalliques en suspension sont mélangées avec une solution contenant un polymère dissout.

Par exemple, dans l'article de Shen et al. (« High dielectric performance of polymer composite films induced by a percolating interparticle barrier layer », Advanced Materials 2007, 19(10), 1418-1422), une fine couche de polymère (PVP) est greffée autour de nanoparticules d'argent par voie hydrothermale. Pour cela, du glucose, du poly (vinylpyrrolidone) (PVP) et de l'AgNO₃ (0,1 M) sont mélangés en solution aqueuse. La solution est ensuite scellée dans un autoclave maintenu à 180°C pendant 4 h, après centrifugation et lavage, les nanoparticules enrobées sont récupérées.

Lorsque la surface de la nanoparticule est en carbone le greffage des polymères est facilité, il se forme aisément des liaisons π- π par transfert d'électrons délocalisés, entre le polymère et la surface de la nanoparticule. Cependant, ces liaisons sont plus faibles que des liaisons covalentes et la stabilité mécanique au cours du temps est moindre.

Afin de former des liaisons covalentes entre un polymère et des nanoparticules, il est possible de réaliser une polymérisation in-situ à partir de monomères. La polymérisation in-situ met en jeu des radicaux libres qui favorisent naturellement la formation de liaisons covalentes. Une telle polymérisation est, par exemple, décrite dans l'article de Herman et al. ("Core double-shell cobalt/graphene/polystyrene magnetic nanocomposites synthesized by in situ sonochemical polymerization", RSC Advances (2015), 5(63), 51371-51381). Des nanoparticules de cobalt recouvertes de graphène en multicouches sont mélangées avec du styrène et un initiateur de polymérisation. La polymérisation est réalisée en présence d'ultra-sons (i.e. par sonochimie) avec une puissance de 100W.

Cependant, afin de réaliser le processus de polymérisation, il est nécessaire d'apporter une grande quantité d'énergie de cavitation dans un mélange homogène de nanoparticules et de monomères. Ce procédé peut conduire à l'endommagement des nanoparticules, en particulier à une détérioration de la coquille de graphène et/ou à l'oxydation du graphène et/ou du coeur métallique des nanoparticules qui n'est plus complètement protégé par le graphène.

De plus, la taille finale des nanoparticules hybrides métal/polymère dépend de la taille initiale des nanoparticules de départ. Cependant, souvent les poudres présentent une grande dispersion en taille qui peut aller de moins de 5nm à plus de 300nm. Or, il est important de pouvoir fournir des nanoparticules hybrides métal/polymère monodispersées puisque les propriétés des nanoparticules sont souvent liées à leur taille. Par exemple, dans le domaine des capacités, les matériaux composites nanodiélectriques sont sujets à des champs de claquage incontrôlés lorsque la taille des nanoparticules est trop dispersée. Dans le domaine des inductances, les nanoparticules trop petites sont sans effet (non magnétiques à l'ambiante) et les très grosses causent des pertes ohmiques à haute fréquence (« eddy current » en anglais).

### EXPOSÉ DE L'INVENTION

Un but de la présente invention est de proposer un procédé de fabrication de nanoparticules comprenant un coeur métallique recouvert par une couche de matériau polymère d'épaisseur déterminée, présentant une faible distribution de taille, la couche de matériau polymère devant présenter une bonne tenue mécanique dans le temps.

Pour cela, la présente invention propose un procédé de fabrication de nanoparticules comprenant un coeur métallique recouvert par une couche de matériau polymère, le procédé comprenant les étapes suivantes :
a) préparation d'une émulsion eau-dans-huile comprenant des gouttelettes d'une phase aqueuse, dispersées dans une phase organique,
b) ajout de nanoparticules comprenant un coeur métallique recouvert par une coquille de matériau carboné, moyennant quoi on obtient des nanoparticules piégées dans les gouttelettes,
c) ajout de monomères précurseurs du matériau polymère, et
d) ajout d'un initiateur de polymérisation.

La mise en présence des monomères et de l'initiateur de polymérisation conduit à la polymérisation des monomères, moyennant quoi on obtient des nanoparticules recouvertes par une couche de matériau polymère, dispersées dans la phase organique.

L'invention se distingue fondamentalement de l'art antérieur par la formation in-situ d'une couche de polymère d'épaisseur contrôlée autour d'une nanoparticule de dimension déterminée dans une émulsion.

Par émulsion, on entend un mélange hétérogène de deux liquides non miscibles, comme l'huile et l'eau. Une des deux phases (dite phase dispersée, ici la phase aqueuse) est dispersée sous forme de gouttelettes dans l'autre (dite phase dispersante). La taille des gouttelettes peut aller de quelques dizaines de nanomètres à plusieurs microns.

Les gouttelettes jouent à la fois le rôle de filtre et le rôle de réacteur de polymérisation.

Seules les nanoparticules dont le diamètre est inférieur au diamètre des gouttelettes seront piégées dans les gouttelettes et enrobées par une couche de polymère au cours du procédé.

Les nanoparticules dont le diamètre est supérieur ou égal à celui des gouttelettes formeront un sédiment brut (sans polymère) qui sera facilement éliminé par la suite. Par ailleurs, il a été observé que les nanoparticules les plus petites (par exemple d'un diamètre de moins de 10nm) sont imparfaitement recouvertes par la couche carbonée et s'oxydent rapidement, ce qui leur confère un caractère plus hydrophobe que les nanoparticules complètement recouvertes par une couche carbonée. Ces particules ne pénètrent pas dans les gouttelettes contenant la phase aqueuse et restent en suspension dans la phase dispersante et pourront être facilement éliminées ultérieurement.

Les gouttelettes forment des micelles qui sont des microréacteurs à l'intérieur desquels a lieu la réaction de polymérisation. Lors de la réaction de polymérisation, les monomères vont être progressivement consommés jusqu'à saturation de la micelle. On parle de croissance micellaire. La taille de la particule finale ainsi formée est semblable à celle de la micelle. On obtient au final un produit appelé latex comprenant des billes monodispersées de polymère (micelles sans nanoparticule métallique) et de nanoparticules hybrides métal-polymère (micelles avec nanoparticules métalliques).

Les nanoparticules métalliques sont recouvertes d'une couche de matériau carboné (nanoparticules métal/C avec C représentant la couche de matériau carboné), pour protéger efficacement les nanoparticules contre l'oxydation et leur conférer des propriétés hydrophiles. Les nanoparticules peuvent ainsi pénétrer dans les gouttelettes contenant la phase aqueuse. La couche carbonée forme, avantageusement, une couche continue autour de chaque nanoparticule. Sans cette coquille carbonée, les nanoparticules métalliques s'oxyderaient spontanément au contact de l'eau et/ou formeraient des hydroxydes métalliques, ce qui les rendrait hydrophobes et contribuerait à les chasser des micelles pour rejoindre la phase dispersante (huile).

Avantageusement, le matériau carboné est du carbone 2D organisé. De préférence, le matériau carboné est du graphène. La couche carbonée est constituée d'une répétition de quelques couches à quelques dizaines de couches de graphène pour conserver une structure organisée 2D du carbone. Par exemple elle comprend quelques couches de graphène (de 2 à 5 par exemple). On choisira, avantageusement, une couche carbonée comprenant au moins deux couches de graphène pour conférer des propriétés hydrophiles suffisantes à la nanoparticule et pour pouvoir former des liaisons de Van der Waals. On choisira, avantageusement, un nombre de couches inférieur à 100, pour éviter l'apparition de défauts dans les couches et d'avoir un arrangement de carbone 2D désorganisé en surface, par exemple hydrogéné, ou de type graphitique qui sont hydrophobes.

Avantageusement, le coeur des nanoparticules est en cobalt, en fer, en nickel, en cuivre, en argent, en or, ou en un de leurs alliages, ou en un de leurs nitrures. Par exemple, on choisira des alliages de cobalt et de fer (CoFe), de cobalt, de fer et de nickel (CoFeNi), de nickel et de fer (NiFe) ou des nitrures de fer Fe₄N ou Fe₁₆N₂.

Avantageusement, le matériau polymère est choisi parmi le polystyrène, le polyméthacrylate de méthyle, le polyuréthane, un polyacrylique, le polypropylène, un polyimide, le polyétherimide et un polymère ayant des groupes pyrène

Avantageusement, l'épaisseur de la couche en matériau polymère va 1nm à 100nm, de préférence de 2nm à 50nm, et encore plus préférentiellement de 5nm à 15nm.

Avantageusement, le diamètre des gouttelettes va de 20nm à 1µm. La taille moyenne des micelles est facilement adaptable à celles des nanoparticules recherchées. On choisira, avantageusement, des micelles ayant une plus grande dimension légèrement supérieure à celle des nanoparticules que l'on souhaite piéger pour pouvoir introduire sélectivement ces dernières dans les micelles sans déstabiliser l'émulsion.

Avantageusement, le diamètre des gouttelettes va de 30nm à 100nm.

Avantageusement, des nanoparticules électriquement isolantes sont ajoutées à l'émulsion. Les particules sont, avantageusement, hydrophobes de manière à être dispersées dans la phase organique avec les monomères. Lors de la polymérisation, une partie de ces nanoparticules électriquement isolantes sont entrainées par les monomères et se retrouvent piégées dans et/ou sur la couche de polymère enrobant la nanoparticule métallique.

Avantageusement, les nanoparticules électriquement isolantes sont en oxyde métallique (par exemple SiO₂, Al₂O₃, TiO₂, HfO₂, Ta₂O₅, BaTiO₃ ou SrTiO₃). Encore plus avantageusement, les nanoparticules électriquement isolantes sont en un matériau semi-conducteur, de préférence à grand gap, comme le carbure de silicium, le diamant et/ou le nitrure de bore hexagonal. Dans le cas du nitrure de bore hexagonal, il peut s'agir également de nanotubes et/ou de nanofeuillets.

Le procédé de polymérisation en milieu dispersé présente de nombreux avantages par rapport à la polymérisation classique en milieu homogène. Notamment, il permet de :
- greffer de manière covalente un polymère sur des nanoparticules métalliques sans oxydation significative des nanoparticules qui conservent leurs propriétés initiales (par exemple magnétiques et/ou électriques),
- contrôler plus finement l'épaisseur du polymère (par exemple 5, 10 ou 15nm) par rapport au greffage dit ex-situ d'un polymère préexistant mélangé aux nanoparticules,
- disperser de manière homogène des nanoparticules dans l'émulsion,
- limiter voire supprimer la présence d'agrégats,
- contrôler facilement de la température de réaction,
- diminuer la viscosité du milieu réactionnel (principal problème avec les polymères),
- obtenir un matériau composite prêt à l'emploi (colloïde ou poudre).

De plus, le procédé est simple à mettre en oeuvre et nécessite peu de matière première.

Une poudre de nanoparticules obtenue par le procédé défini précédemment est décrite sans être revendiquée, les nanoparticules comprenant un coeur métallique recouvert successivement par une couche de matériau carboné et par une couche en un matériau polymère, les nanoparticules présentent une faible polydispersité (i.e. une faible distribution de taille).

Par faible polydispersité, on entend l'absence d'agrégats ou de très grandes nanoparticules (par exemple ayant une plus grande taille, ou plus grande dimension, jusqu'à 100 fois, 30 fois ou 10 fois plus grande que la taille moyenne des nanoparticules) et l'absence de très petites particules (ayant une taille, par exemple, jusqu'à 6 fois ou 10 fois plus petite que la taille moyenne des nanoparticules). Le rapport entre le diamètre maximal des nanoparticules et le diamètre minimal des nanoparticules est inférieur ou égal à 5, de préférence inférieur ou égal à 3.

Avantageusement, le rapport diamètre maximal/diamètre minimal des nanoparticules est inférieur ou égal à 2 et encore plus avantageusement inférieur ou égal à 1,5. Une telle distribution de taille, très resserrée, est rendue possible par le procédé de mini-émulsion décrit précédemment.

La polymérisation en milieu hétérogène nécessite moins de radicaux et moins d'oxydant. Avantageusement, les nanoparticules, par exemple, ayant un coeur en cobalt ou en fer, obtenues avec ce procédé, présentent des propriétés magnétiques, elles ont une aimantation supérieure ou égale à 100emu/g.

L'utilisation d'une poudre de nanoparticules telle que définie précédemment, pour réaliser une inductance, un filtre ou une capacité est également décrite sans être revendiquée. Par exemple, il s'agit d'une inductance, d'un filtre (passe-bas, passe-haut, passe-bande, coupe bande ou filtre de mode commun) ou d'une capacité en couches minces sur un substrat, de préférence, en silicium. D'autres substrats peuvent être utilisés comme le verre, l'alumine, le ferrite et les films polymères comme par exemple le polyimide (tel que le Kapton), le poly(naphtalate d'éthylène), et le poly(téréphtalate d'éthylène) (PET).

Un dispositif électronique tel qu'une inductance, un filtre ou une capacité, par exemple une inductance, un filtre ou une capacité en couches minces sur un substrat, de préférence, en silicium, comprenant des nanoparticules, telles que définies précédemment, dispersées dans une matrice est également décrit sans être revendiqué. L'utilisation de telles particules, par exemple, dans une capacité permet d'augmenter significativement la permittivité.

La matrice est, avantageusement, une matrice minérale ou polymère.

Avantageusement, la matrice est en un matériau polymère choisi parmi le polyéthylène téréphtalate, l'acétate de cellulose, le polycarbonate, le polypropylène, le polyéthylène, un polyamide, un polysiloxane, un polysulfone, un polyester éventuellement aromatique, un polyétheréthercétone, un polyétherimide et un époxyde.

Avantageusement, le matériau polymère comprend des groupements photosensibles aux rayons ultra-violets.

Avantageusement, la matrice comporte, en outre, des nanoparticules électriquement isolantes.

D'autres caractéristiques et avantages de l'invention ressortiront du complément de description qui suit.

Il va de soi que ce complément de description n'est donné qu'à titre d'illustration de l'objet de l'invention et ne doit en aucun cas être interprété comme une limitation de cet objet.

### BRÈVE DESCRIPTION DES DESSINS

La présente invention sera mieux comprise à la lecture de la description d'exemples de réalisation donnés à titre purement indicatif et nullement limitatif en faisant référence aux dessins annexés sur lesquels :
Les figures 1, 2, 3, 4 et 5 représentent différentes étapes d'un mode de réalisation particulier du procédé de l'invention,
La figure 6 est un graphique représentant la taille moyenne des micelles obtenue par diffusion dynamique de la lumière (DLS) en fonction du pourcentage (en poids) d'additifs dans la phase aqueuse,
Les figures 7 et 8 représentent, de manière schématique, et en coupe, des nanoparticules avant et après enrobage par une couche de polymère, selon des modes de réalisation particulier de l'invention,
La figure 9 est un cliché photographique montrant une dispersion comprenant un solvant et des nanoparticules métal/C (à gauche) et une émulsion stabilisée (à droite) selon un mode de réalisation particulier du procédé de l'invention,
La figure 10 est un cliché photographique montrant une suspension uniforme de nanoparticules métal/C enrobées par une fine couche de polymère, obtenue selon un mode de réalisation particulier de l'invention,
La figure 11 est un cliché obtenu par microscopie électronique à transmission de nanoparticules à structure coeur/coquille obtenue selon un mode de réalisation particulier du procédé de l'invention,

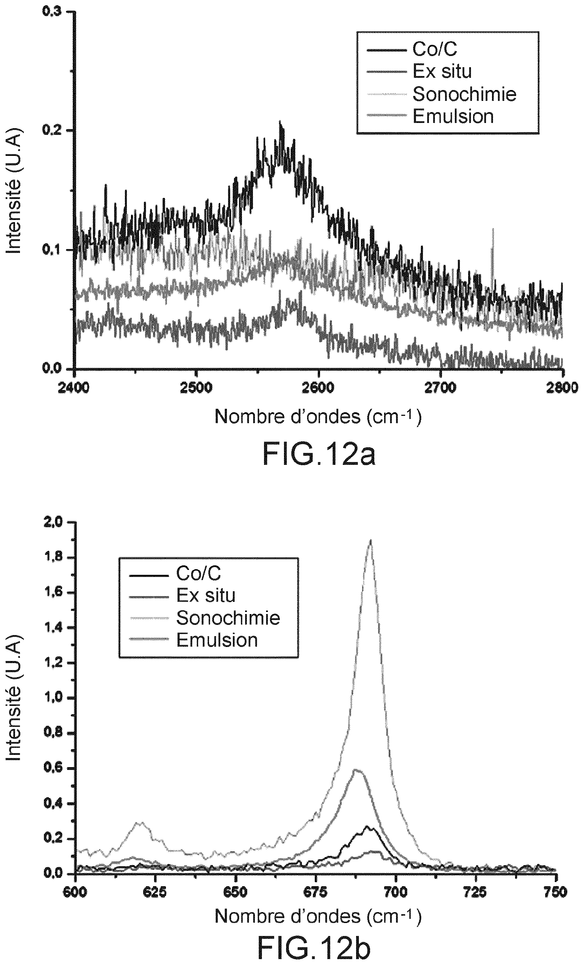

Les figures 12a et12b sont des spectres Raman centrés, respectivement, sur la principale raie du graphène et sur la principale raie de l'oxyde de cobalt, obtenus sur des nanoparticules Co/C, des nanoparticules Co/C recouvertes par une couche de polymère selon une méthode de greffage non-covalent en milieu homogène avec polymère préexistant (désignée par 'ex-situ'), des nanoparticules Co/C recouvertes par une couche de polymère selon une méthode de greffage covalent avec polymérisation in-situ en milieu homogène par sonochimie (désignée par 'sonochimie'), des nanoparticules Co/C recouvertes par une couche de polymère selon un mode de réalisation particulier du procédé de l'invention, soit une méthode de greffage covalent avec polymérisation in-situ en milieu hétérogène ou mini-émulsion (désigné par 'émulsion').

Les différentes parties représentées sur les figures ne le sont pas nécessairement selon une échelle uniforme, pour rendre les figures plus lisibles.

Les différentes possibilités (variantes et modes de réalisation) doivent être comprises comme n'étant pas exclusives les unes des autres et peuvent se combiner entre elles.

### EXPOSÉ DÉTAILLÉ DE MODES DE RÉALISATION PARTICULIERS

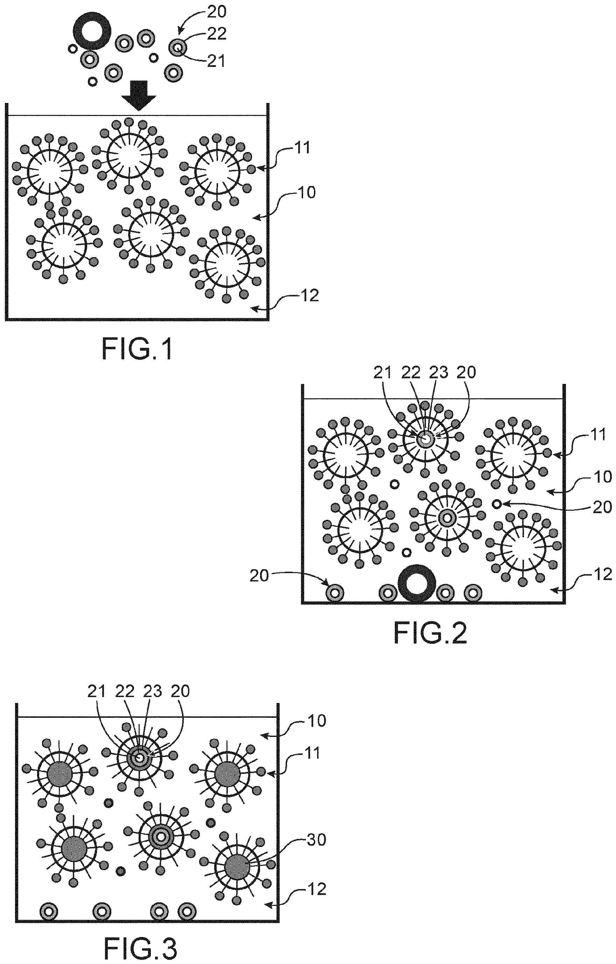

On se réfère, en premier, aux figures 1 à 5 qui représentent les différentes étapes d'un procédé pour enrober des nanoparticules 20 d'une couche de polymère 23 d'épaisseur contrôlée. Le procédé comprend les étapes suivantes :
a) préparation d'une émulsion 10 eau-dans-huile comprenant des gouttelettes 11 d'une phase aqueuse, dispersées dans une phase organique 12 (figure 1),
b) ajout de nanoparticules 20 comprenant un coeur métallique 21 recouvert par une coquille de matériau carboné 22, moyennant quoi on obtient des nanoparticules 20 piégées dans les gouttelettes 11 (figure 2),
c) ajout de monomères précurseurs du matériau polymère, et
d) ajout d'un initiateur de polymérisation.

La mise en présence des monomères et de l'initiateur de polymérisation conduit à la polymérisation des monomères, moyennant quoi on obtient des nanoparticules 20 recouvertes par une couche de matériau polymère 23, dispersées dans la phase organique 12 (figures 3, 4 et 5).

Lors de l'étape a), on met en présence une phase aqueuse et une phase organique de manière à obtenir un mélange bi-phasique, puis on réalise une émulsification du mélange bi-phasique en présence d'un tensio-actif (ou émulsifiant), moyennant quoi on forme une émulsion 10 eau-dans-huile, formée de gouttelettes 11 de la phase aqueuse dispersée dans la phase organique 12. Les gouttelettes 11 forment des micelles (coeur hydrophile - queues hydrophobes).

L'émulsification est, par exemple, formée par agitation (ultrasons). Le mélange reste stable grâce à l'ajout de l'émulsifiant. La vitesse ou la cinétique d'évolution du mélange est quasi nulle, ce qui en fait un milieu réactionnel confiné particulièrement stable et propice à la synthèse de polymères par polymérisation de monomères.

L'émulsion 10 peut contenir d'autres ingrédients, non réactifs, mais nécessaires à la stabilisation de l'émulsion.

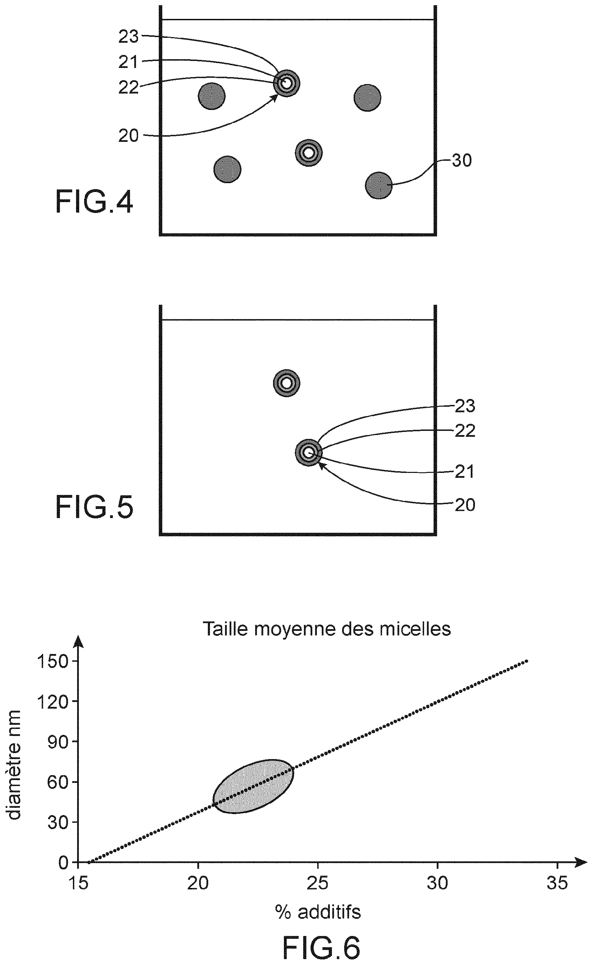

Comme représenté sur la figure 6, la taille des micelles 11 varie en fonction de la quantité de tensio-actifs (additifs) présente dans l'émulsion. En revanche, la taille des micelles 11 ne change pas avec l'ajout des nanoparticules.

Par taille, on entend ici et par la suite, la taille moyenne.

De préférence, l'émulsion 10 est une mini-émulsion, c'est-à-dire les gouttelettes 11 dispersées, dans la phase organique 12, présentent une taille allant de 20nm à 1µm, de préférence de 30nm à 100nm, et plus préférentiellement de 30nm à 60nm. On choisira la taille des gouttelettes 11 en fonction de la taille des nanoparticules 20 et de l'épaisseur de la couche de polymère 23 désirée.

Lors de l'étape b), des nanoparticules 20 sont ajoutées à l'émulsion précédemment formée.

Par nanoparticules, on entend des éléments de taille submicronique (typiquement inférieure à 1µm) de forme, par exemple, sphériques, allongées, ovoïdes. De préférence, il s'agit de particules sphériques. On appelle diamètre ou taille leur plus grande dimension.

Elles peuvent présenter une forte distribution de taille, par exemple avoir un diamètre allant de 5nm à 1µm. Cette taille peut être déterminée par spectroscopie à corrélation de photons.

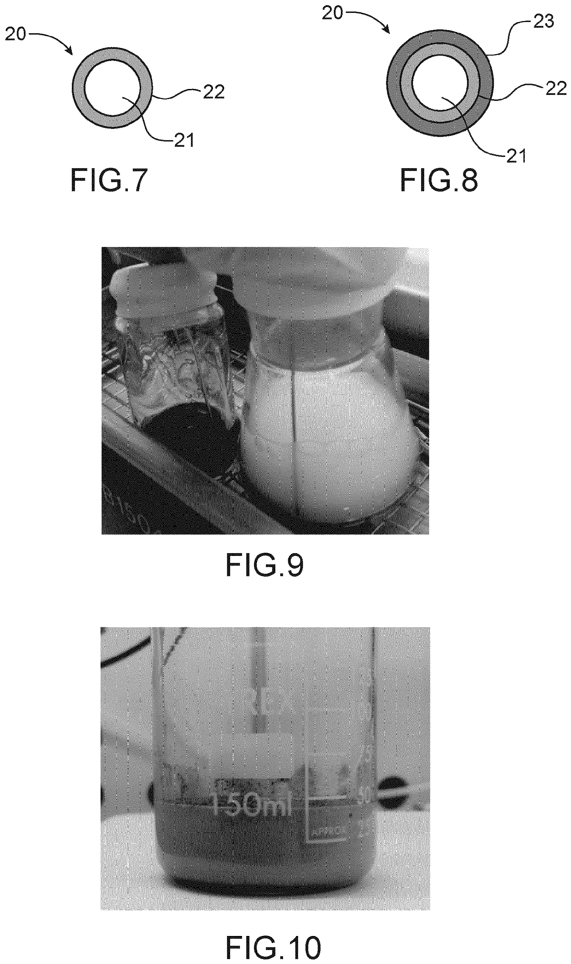

Comme représenté sur la figure 7, les particules 20 ont une structure coeur-coquille. La coquille 22 est solidaire du coeur 21 de la particule.

Le coeur 21, ou noyau, est un matériau métallique. Par matériau métallique, on entend un métal ou un alliage de métal. De préférence, il s'agit d'un métal. De préférence, il s'agit de cobalt, nickel, fer, cuivre, argent ou or. Il peut également s'agir d'un de leurs alliages, comme les alliages de cobalt et de fer (CoFe), de cobalt, de fer et de nickel (CoFeNi), de nickel et de fer (NiFe) ou d'un de leurs nitrures, comme le nitrure de fer Fe₄N ou Fe₁₆N₂.

Le coeur 21 est recouvert par un revêtement 22 ou coquille. Le revêtement 22 est en un matériau carboné organique ou inorganique. De préférence, il s'agit d'un revêtement 22 inorganique.

Le revêtement est un revêtement de carbone 2D organisé sur une surface non plane (par exemple sur la surface d'une nanoparticule).

De préférence, le revêtement 22 est en graphène. Il peut comprendre une couche ou plusieurs (deux, trois, quatre,...) couches de graphène. Par exemple, il comprend de 1 à 50 feuillets de graphène, de préférence de 2 à 10, par exemple de 2 à 5, et encore plus préférentiellement de 3 à 10.

De préférence, la coquille 22 carbonée est continue de manière à recouvrir complètement le coeur 21 de la particule 20 pour protéger le coeur des nanoparticules de l'oxydation, et les rendre plus hydrophiles.

Les nanoparticules 20 ajoutées à l'étape b) sont notées nanoparticules métal/C.

Les nanoparticules 20 peuvent être fabriquées par pyrolyse d'aérosol (SP pour « spray pyrolysis ») par flamme, par laser ou par plasma ou par dépôt chimique en phase vapeur (CVD). Ce type de poudre présente une forte distribution de taille.

La coquille carbonée 22 peut être fabriquée par décomposition d'un gaz précurseur contenant du carbone, par exemple l'acétylène, par SP ou CVD.

L'émulsion va permettre de trier ces nanoparticules 20 en fonction de leur taille. Par exemple, pour une poudre dont le diamètre moyen des nanoparticules 20 est de l'ordre de 30 nm, la dispersion en taille est grande et peut aller de moins de 5nm à plus de 300nm. Cela est préjudiciable pour la fabrication d'un matériau nanocomposite à propriétés contrôlées.

De préférence, la taille des micelles 11 est 2 à 3 fois supérieure à la taille moyenne des nanoparticules 20 (par exemple entre 60 et 90 nm pour 30 nm). Seules les nanoparticules 20 de taille moyenne de l'ordre de 30 nm seront piégées dans l'émulsion et seules ces nanoparticules 20 seront ultérieurement enrobées d'une couche de polymère 23. De cette manière, les nanoparticules 20 les plus grosses (par exemple 100 nm ou 300 nm) formeront un sédiment non recouvert de polymère qui sera facilement éliminé par la suite. Les nanoparticules 20 les petites (par exemple de 10 nm et moins) imparfaitement recouvertes de revêtement carboné 22 s'oxydent rapidement (formation de carboxyles et/ou d'oxydes métalliques), et donc plus hydrophobes, restent dans la phase organique (figures 1 à 4).

Comme représenté sur la figure 2, environ un tiers des nanoparticules 20 de diamètre d'intérêt sont stabilisées dans les micelles 11. Les autres nanoparticules 20, sont en suspension dans la phase organique ou dans le sédiment, et ne participent pas à la polymérisation.

En fonction de la taille des nanoparticules 20, une micelle 11 peut contenir une seule nanoparticule 20 ou plusieurs nanoparticules 20, par exemple sous la forme d'agrégat. De manière avantageuse, une micelle 11 contient une seule nanoparticule 20.

Selon une variante du procédé de réalisation, non représentée, l'émulsion 10 comporte, en outre, des éléments hydrophobes. Les éléments hydrophobes sont dispersés dans la phase dispersante (i.e. la phase organique). De préférence, les éléments sont électriquement isolants. Par électriquement isolant, on entend une résistivité électrique intrinsèque supérieure à 10¹² ohm.cm.

Il peut s'agir de nanoparticules minérales, par exemple des nanoparticules de silice, des nanoparticules d'oxydes complexes, par exemple de titanate de baryum (BaTiO₃) ou/et strontium (SrTiO₃), des nanoparticules de diamant et/ou des nanoparticules de carbure de silicium (SiC).

Il peut s'agir de nanoparticules tubulaires ou lamellaires.

Par nanoparticules tubulaires ou lamellaires, on entend des particules dont l'une des dimensions est très inférieure aux deux autres. De telles particules tubulaires ou lamellaires comportent le plus souvent une épaisseur e (ou un diamètre d) bien inférieure à leur longueur L ou largeur l. De préférence, le rapport e/L (ou d/L) et e/l (ou d/l) est inférieur ou égal à 0,5 et de préférence inférieur ou égal à 0,1 ou à 0.01.

Avantageusement, les nanoparticules tubulaires ou lamellaires sont en nitrure de bore hexagonal (h-BN). Il peut également s'agir d'oxyde de graphène GO.

Les nanoparticules lamellaires peuvent, par exemple, être exfoliées. Par exfolié, on entend que l'on retire des lamelles ou feuilles de l'empilement formant les nanoparticules lamellaires de manière à obtenir des particules lamellaires formées d'une ou de quelques feuilles (2, 3, 4 ou 5 par exemple). Les nanoparticules tubulaires peuvent être fabriquées par décomposition de gaz précurseur par SP ou par CVD.

On ajoute dans l'émulsion 10, formée à l'étape b), l'initiateur de polymérisation et les monomères, précurseurs du polymère. Avantageusement, on choisit des quantités d'initiateur et de monomère de manière à obtenir un rendement de polymérisation faible afin de créer une couche 23 très fine de polymère à la surface des nanoparticules 20 (par exemple de 5nm à 10 nm). Par faible, on entend un rendement de polymérisation inférieur à 50%, et de préférence inférieur à 25%, préférentiellement inférieur à 20%, par exemple de l'ordre de 10%. La quantité de monomères consommée est fixée par le rendement de polymérisation.

Avantageusement, le polymère est choisi parmi le polystyrène (PS), le polyméthacrylate de méthyle (PMMA), le polyuréthane (PU), un polyacrylique (PAA), le polypropylène (PP), un polyimide (PI) et le polyétherimide (PEI). Le polymère peut également être un polymère fonctionnalisé par un groupement pi conjugué, tel que le pyrène. Il s'agit, par exemple, de polystyrène fonctionnalisé par un groupement pyrène (Py-PS) ou encore d'un polyacrylique fonctionnalisé par un groupement pyrène (Py-PAA).

Selon une première variante de réalisation, les étapes c) et d) sont réalisées simultanément.

Selon une autre variante de réalisation, le procédé comporte successivement les étapes c), d) et c).

Selon un autre mode de réalisation, l'étape c) est réalisée préalablement à l'étape b).

Lorsque les précurseurs du polymère sont mis en présence de l'initiateur de polymérisation (aussi appelé amorceur de polymérisation), on initie la polymérisation (figure 3).

La polymérisation est une polymérisation radicalaire. Celle-ci est amorcée par l'entrée dans la micelle 11 d'un oligo-radical (hydrophile) préalablement formé en phase aqueuse qui va engendrer une consommation progressive des monomères (hydrophobes) stockés dans la phase dispersante (ici l'huile), jusqu'à saturation de la micelle.

On se place dans des conditions aptes à faire réagir l'amorceur de polymérisation, typiquement par élévation de la température et/ou par sonication.

Par exemple, la polymérisation est effectuée par chauffage de l'émulsion à une température comprise de 40°C à 80°C, de préférence de 50°C à 80°C, et préférentiellement de 60°C à 70°C. Ces gammes de température peuvent être adaptées en fonction de la température à laquelle l'amorceur de polymérisation devient réactif.

L'étape de polymérisation dure généralement de quelques minutes à quelques dizaines de minutes, par exemple environ 20 minutes. Cette étape peut être effectuée sous ultrasons à l'aide d'une sonde de sonication.

A l'issue de l'étape de polymérisation (figure 4), les gouttelettes 11 de l'émulsion 10 sont converties en éléments solides dispersés dans la phase organique. Par « dispersion », on entend une suspension stable d'éléments solides, de préférence individualisés et non agglomérés, dans une phase continue liquide. Les éléments ont une taille moyenne équivalente à la taille moyenne des gouttelettes 11 de l'émulsion 10 dont ils sont issus.

Les éléments solides peuvent être des billes 30 de matériau polymère, obtenues dans le cas où les micelles ne contiennent pas, au moment de la polymérisation, de nanoparticules 20 métal/C. Alternativement, comme représenté sur la figure 8, les éléments solides peuvent être des nanoparticules 20 métal/C enrobée d'une couche de polymère 23, notées métal/C/polymère, dans le cas où les micelles contiennent, au moment de la polymérisation, des nanoparticules 20 métal/C.

Dans le cas où une micelle 11 comporte, au début de l'étape de polymérisation, plusieurs nanoparticules 20, la couche de polymère 23 enrobe l'ensemble de ces nanoparticules 20.

Le procédé permet de greffer de manière covalente une couche 23 de polymère à la surface des nanoparticules 20.

Le procédé est réalisé à pression ambiante (1bar) dans une enceinte hermétique, par exemple en verre. Le procédé est, avantageusement, réalisé avec un bullage d'azote pour désoxygéner le milieu réactionnel

A l'issue de l'étape de polymérisation, on réalise, avantageusement, un « lavage » (séquence de précipitation/dilution) pour éliminer les produits de réaction non consommés et récupérer le latex. Le latex, semblable à une poudre cohésive, est constitué de nanoparticules 20 métal/C/polymère, de taille très homogène (par exemple 40 nm ± 2 nm), et de billes 30 de polymère (figure 4).

Une étape de séparation par centrifugation peut ensuite être réalisée pour séparer les nanoparticules métal/C/polymère des billes de polymère (figure 5). On peut ainsi récupérer environ un tiers des nanoparticules 20 introduites.

Les nanoparticules 20 obtenues avec le procédé forment une poudre. Les nanoparticules sont monodispersées, c'est-à-dire qu'elles ont une distribution de taille comprise entre un diamètre maximal et un diamètre minimal tel que leur rapport soit inférieur ou égal à 5, 3 ou 2 et avantageusement inférieur ou égal à 1,5 par exemple 1,3 ou 1,2 ou 1,1. Les caractéristiques d'une telle poudre (rapport 1,1) sont, par exemple, un diamètre moyen des nanoparticules de 40nm, un diamètre maximal de 42nm et un diamètre minimal de 38nm. Le diamètre des nanoparticules 20 peut être mesuré avec un granulomètre laser ou par diffraction de la lumière (DLS pour « Dynamic Light Scattering ») en solution.

Toutes les caractéristiques dimensionnelles mentionnées précédemment et par la suite peuvent être également mesurées par les techniques suivantes : MEB (microscope électronique à balayage) et TEM (microscope électronique en transmission), ellipsométrie et spectrophotométrie.

Les nanoparticules 20 métal/C/polymère peuvent ensuite être utilisées pour réaliser un autre matériau. Par exemple, elles peuvent être dispersées dans un solvant et former une solution colloïdale stable. Elles peuvent être dispersées dans une matrice minérale ou dans une matrice polymère, le polymère étant identique ou différent de celui de la coquille. Il peut s'agir d'un polymère thermoplastique ou d'une résine photosensible. A titre illustratif, elles peuvent être enrobées dans une matrice polymère (par exemple en polystyrène, en époxy ou en polyimide) de manière à former un film nanocomposite métal-polymère uniforme.

La matrice polymère, par exemple en PI ou en époxyde, peut contenir des agents de réticulation photosensibles, de préférence aux ultra-violets (UV).

Ce film peut être formé, sur un substrat, par exemple en silicium, par dépôt à la tournette (« spincoating »). D'autres techniques de dépôt peuvent être envisagées, telle que l'enduction par immersion (« dip coating »), la sérigraphie (« screen printing ») ou le jet d'encre (« ink jet »). On choisira une technique de dépôt dont les températures mises en jeu ne dépassent pas la température de fusion de la coquille polymère de la nanoparticule.

Le film peut être exposé à une source lumineuse, de préférence à une source ultra-violette (UV). Le film peut être pressé à chaud, de préférence au voisinage de la température de transition vitreuse.

Un tel film contient une dispersion homogène de nanoparticules métalliques faiblement dispersées en taille. De plus, comme les nanoparticules sont solidement enrobées par une fine couche régulière de polymère diélectrique, la distance de séparation entre les nanoparticules est maitrisée. Par exemple, la distance de séparation bord à bord entre deux particules métal/C de diamètre φ est de 1 × φ, 1/ 2 × φ ou 2/3 × φ et avantageusement inférieure ou égale à 1/3 × φ. Par exemple, un tel film est caractérisé par une dispersion homogène de nanoparticules de Co/C de diamètre moyen 30nm avec une distance intergranulaire moyenne de 10nm, jusqu'à 5nm.

Le pourcentage volumique de nanoparticules dans le matériau composite va, par exemple, de 0,01 à 30%.

Avantageusement, pour former une capacité, le pourcentage volumique va par exemple de 0,01% à 10 %, par exemple de 0,01% à 5 %, et de préférence de 0,1% à 2%.

Avantageusement, pour les inductances ou les filtres, le pourcentage volumique est supérieur à 10%, par exemple de 10% à 30%, voire supérieur à 30%. Le pourcentage massique de nanoparticules dans le matériau composite va, par exemple, de 0,5% à 90 %, et de préférence de 0,5% à 10% pour les capacités et de 50 % à 90 % pour les inductances ou les filtres.

Un tel film présente une grande résistivité (par exemple 10¹²-10¹⁵µOhm.cm).

Un tel matériau composite peut, par exemple, former un élément de dispositif électronique comme une inductance, un filtre ou une capacité, et plus particulièrement une inductance ou un filtre en couches minces sur silicium ou sur ferrite ou une capacité en couches minces sur silicium. Une telle inductance et un tel filtre peuvent servir pour l'intégration des modules de conversion d'énergie ou de filtrage RF, par exemple pour la pour la téléphonie 5G. Une telle capacité peut servir pour l'intégration des modules de conversion de puissance, par exemple pour les véhicules électriques.

### Exemples illustratifs et non limitatifs d'un mode de réalisation

*Nanoparticules utilisées : Co*/*C, diamètre 30-50 nm*
*Produits et conditions utilisés pour la polymérisation in-situ :*
   - Monomère : Styrène 99%,
   - Surfactant : déoxycholate de sodium (DOC) 97%,
   - Initiateur : 2, 2'- azobis (2-methyl propionitrile) (AIBN) 98%,
   - Proportion : DOC + AIBN = 1% Styrène (en poids),
   - Température de réaction = 65°C,
   - Bullage N₂,
   - Sonication : 500 W à 20 KHz,
   - Durée : 20 à 30 min.

*Produits et conditions utilisés pour récupérer le latex (précipitation*/*lavage) :*
- Produit d'émulsion (latex brut, résidus monomères, initiateur, surfactant),
- Solvant pour précipitation : méthanol 98%,
- Solvant pour dilution (Styrène) : toluène 98%,
- Séquence précipitation/dilution répétée 4 fois,
- Séchage à l'air sous vide.

*Produits et conditions utilisés pour récupérer les nanoparticules enrobées de polymère :*
- Latex lavé (nanoparticules enrobées et nano-billes de polymère),
- Solvant : chloroforme 98%,
- Agitation + Centrifugation à 12 10³ tour/min pendant 20 min,
- Récupération du surnageant,
- Séquence répétée 4 fois.

*Produits et conditions utilisés pour le dépôt d'un film comprenant les nanoparticules enrobées de polymère :*
- Particules enrobées,
- Solvant : chloroforme 98%,
- Ajout du polymère support : Polystyrène 0,75 g/mL (35 kg/mol),
- Sonication : 500 W à 20 kHz,
- Durée : 20 min,
- Spin-coating : 1000 tour/min,
- Séchage sur plaque chauffante à 65°C pendant 10 min.

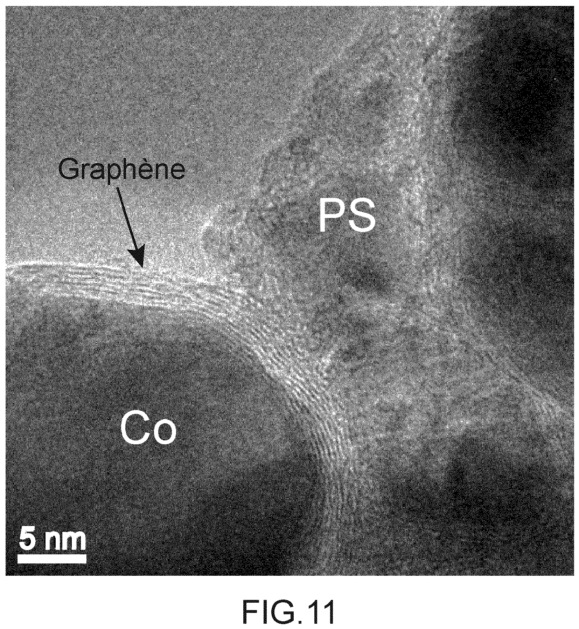

L'épaisseur de polymère greffé à la surface des nanoparticules Co/C est de l'ordre de 5 nm (figure 11).

Les spectres Raman obtenus sur ces nanoparticules 20 montrent que la couche de carbone n'est pas endommagée et/ou que l'oxydation est contenue, contrairement à d'autres techniques couramment utilisées pour enrober des nanoparticules telle que la polymérisation in-situ en milieu homogène (ou sonochimie), ou telle qu'une technique consistant à enrober des nanoparticules 20 avec un polymère préalablement polymérisé séparément (ou ex situ) (figures 12a et 12b).

Les mêmes résultats ont été observés pour des nanoparticules Ni/C recouvertes par une couche de polystyrène.

## Revendications

1. Procédé de fabrication de nanoparticules (20) comprenant un coeur (21) métallique recouvert par une couche de matériau polymère (23) comprenant les étapes suivantes :
a) préparation d'une émulsion (10) eau-dans-huile comprenant des gouttelettes (11) d'une phase aqueuse, dispersées dans une phase organique (12),
b) ajout de nanoparticules (20) comprenant un coeur métallique (21) recouvert par une coquille de matériau carboné (22), moyennant quoi on obtient des nanoparticules (20) piégées dans les gouttelettes,
c) ajout de monomères précurseurs du matériau polymère, et
d) ajout d'un initiateur de polymérisation,
l'ajout des monomères et de l'initiateur de polymérisation conduisant à la polymérisation des monomères, moyennant quoi on obtient des nanoparticules (20), enrobées par une couche de matériau polymère (23), dispersées dans la phase organique (12).

2. Procédé selon la revendication 1, **caractérisé en ce que** le matériau carboné est du carbone 2D organisé, de préférence du graphène.

3. Procédé selon l'une des revendications précédentes, **caractérisé en ce que** le coeur (21) des nanoparticules est en cobalt, en fer, en nickel, en cuivre, en argent, en or, ou en un de leurs alliages, ou en un de leurs nitrures.

4. Procédé selon l'une quelconque des revendications précédentes, **caractérisé en ce que** le matériau polymère est choisi parmi le polystyrène, le polyméthacrylate de méthyle, le polyuréthane, un polyacrylique, le polypropylène, un polyimide, le polyétherimide et un polymère ayant un groupe pyrène.

5. Procédé selon l'une quelconque des revendications précédentes, **caractérisé en ce que** l'épaisseur de la couche en matériau polymère (23) mesurée par microscopie électronique à balayage va de 1nm à 100nm, de préférence de 2nm à 50nm, et encore plus préférentiellement de 5nm à 15nm.

6. Procédé selon l'une quelconque des revendications précédentes, **caractérisé en ce que** le diamètre des gouttelettes (11) mesuré par diffraction de la lumière va de 20nm à 1µm, et de préférence, de 30nm à 100nm.

7. Procédé selon l'une quelconque des revendications précédentes, **caractérisé en ce que** des nanoparticules électriquement isolantes sont ajoutées à l'émulsion.

8. Procédé selon la revendication précédente, **caractérisé en ce que** les nanoparticules électriquement isolantes sont en oxyde métallique, par exemple en oxyde de baryum et/ou de strontium, en carbure de silicium, en diamant ou en nitrure de bore hexagonal.

## Patentansprüche

1. Verfahren zur Herstellung von Nanopartikeln (20), die einen Metallkern (21) umfassen, der von einer Schicht aus Polymermaterial (23) bedeckt ist, umfassend die folgenden Schritte:
a) Zubereiten einer Wasser-in-Öl-Emulsion (10), die Tröpfchen (11) einer wässrigen Phase umfasst, welche in einer organischen Phase (12) dispergiert sind,
b) Zugeben von Nanopartikeln (20), die einen Metallkern (21) umfassen, der von einer Hülle aus kohlenstoffhaltigem Material (22) bedeckt ist, wodurch Nanopartikel (20) erhalten werden, die in den Tröpfchen eingeschlossen sind,
c) Zugeben von Vorläufermonomeren des Polymermaterials, und
d) Zugeben eines Polymerisationsinitiators,
wobei die Zugabe der Monomere und des Polymerisationsinitiators zur Polymerisation der Monomere führt, wodurch Nanopartikel (20) erhalten werden, die von einer Schicht aus Polymermaterial (23) überzogen und in der organischen Phase (12) dispergiert sind.

2. Verfahren nach Anspruch 1, **dadurch gekennzeichnet, dass** das kohlenstoffhaltige Material organisierter 2D-Kohlenstoff, vorzugsweise Graphen, ist.

3. Verfahren nach einem der vorstehenden Ansprüche, **dadurch gekennzeichnet, dass** der Kern (21) der Nanopartikel aus Kobalt, Eisen, Nickel, Kupfer, Silber, Gold oder einer ihrer Legierungen oder einem ihrer Nitride besteht.

4. Verfahren nach einem der vorstehenden Ansprüche, **dadurch gekennzeichnet, dass** das Polymermaterial ausgewählt ist aus Polystyrol, Polymethylmethacrylat, Polyurethan, einem Polyacryl, Polypropylen, einem Polyimid, Polyetherimid und einem Polymer mit einer Pyren-Gruppe.

5. Verfahren nach einem der vorstehenden Ansprüche, **dadurch gekennzeichnet, dass** die Dicke der Schicht aus Polymermaterial (23), durch Rasterelektronenmikroskopie gemessen, im Bereich von 1 nm bis 100 nm, vorzugsweise 2 nm bis 50 nm und noch bevorzugter 5 nm bis 15 nm liegt.

6. Verfahren nach einem der vorstehenden Ansprüche, **dadurch gekennzeichnet, dass** der Durchmesser der Tröpfchen (11), durch Lichtbeugung gemessen, im Bereich von 20 nm bis 1 µm, und vorzugsweise 30 nm bis 100 nm liegt.

7. Verfahren nach einem der vorstehenden Ansprüche, **dadurch gekennzeichnet, dass** der Emulsion elektrisch isolierende Nanopartikel zugegeben werden.

8. Verfahren nach dem vorstehenden Anspruch, **dadurch gekennzeichnet, dass** die elektrisch isolierenden Nanopartikel aus Metalloxid, zum Beispiel Barium- und/oder Strontiumoxid, Siliziumkarbid, Diamant oder hexagonalem Bornitrid bestehen.

## Claims

1. Method for manufacturing nanoparticles comprising a metallic core (21) coated with a layer of polymer material (23) comprising the following steps:
a) preparing a water-in-oil emulsion (10) comprising droplets (11) of an aqueous phase, dispersed in an organic phase (12),
b) adding nanoparticles (20) comprising a metallic core (21) coated with a shell of carbonaceous material (22), whereby nanoparticles (20) trapped in the droplets are obtained,
c) adding precursor monomers of the polymer material, and
d) adding a polymerisation initiator,
adding the precursor monomers of the polymer material and the polymerisation initiator resulting in polymerisation of the monomers, whereby nanoparticles (20) coated with a layer of polymer material (23), dispersed in the organic phase (12), are obtained.

2. Method according to claim 1, wherein the carbonaceous material is organised 2D carbon, preferably graphene.

3. Method according to any one of the preceding claims, wherein the core (21) of the nanoparticles is made of cobalt, iron, nickel, copper, silver, gold, or of one of the alloys thereof, or of one of the nitrides thereof.

4. Method according to any one of the preceding claims, wherein the polymer material is chosen among polystyrene, poly(methyl methacrylate), polyurethane, a polyacrylic, polypropylene, a polyimide, polyetherimide and a polymer having a pyrene group.

5. Method according to any one of the preceding claims, wherein the thickness of the layer of polymer material (23) measured by scanning electron microscopy ranges from 1 nm to 100 nm, preferably from 2 nm to 50 nm and even more preferably from 5 nm to 15 nm.

6. Method according to any one of the preceding claims, wherein the diameter of the droplets (11) measured by light diffraction ranges from 20 nm to 1 µm and preferably from 30 nm to 100 nm.

7. Method according to any one of the preceding claims, wherein electrically insulating nanoparticles are added to the emulsion.

8. Method according to the preceding claim, wherein the electrically insulating nanoparticles are made of metal oxide, for example of barium and/or strontium oxide, silicon carbide, diamond or hexagonal boron nitride.
